# EUROPEAN PATENT SPECIFICATION

(11) **EP 2 001 492 B1**
(45) Date of publication and mention of the grant of the patent: **12.09.2012**
(21) Application number: 07718606.2
(22) Date of filing: 23.03.2007
(51) Int. Cl.: A61K 31/728, C07K 16/40, A61K 39/395, A61P 35/00

(54) **A METHOD OF TREATING CANCER AND/OR CELLULAR PROLIFERATIVE CONDITIONS AND AGENTS TARGETING HYALURONAN ANABOLISM USEFUL FOR SAME**
VERFAHREN ZUR BEHANDLUNG VON KREBS UND/ODER ZELLPROLIFERATIONSZUSTÄNDEN UND DAFÜR NÜTZLICHE MITTEL MIT HYALURONAN-ANABOLISMUS ALS TARGET
MÉTHODE POUR TRAITER LE CANCER ET/OU DES ÉTATS PROLIFÉRATIFS DE CELLULES ET AGENTS CORRESPONDANTS VISANT L'ANABOLISME DE L'HYALURONANE

(30) Priority: 31.03.2006 AU 2006901708
(43) Date of publication of application: 17.12.2008
(73) Proprietor: Alchemia Oncology Limited, Eight Mile Plains, QLD 4113 (AU)
(72) Inventor: BROWN, Tracey, Flemington, VIC 3031 (AU); BROWNLEE, Gary, Camberwell, Victoria 3124 (AU)
(74) Representative: HOFFMANN EITLE
(86) International application number: PCT/AU2007/000359
(87) International publication number: WO 2007/112475

(56) References cited:
- EP-A2- 0 881 294
- WO-A-2005/035548
- WO-A1-2005/035548
- UDABAGE ET AL: "The over-expression of HAS2, Hyal-2 and CD44 is implicated in the invasiveness of breast cancer" EXPERIMENTAL CELL RESEARCH, ACADEMIC PRESS, US, vol. 310, no. 1, 15 October 2005 (2005-10-15), pages 205-217, XP005093320 ISSN: 0014-4827
- UDABAGE LISHANTHI ET AL: "Antisense-mediated suppression of hyaluronan synthase 2 inhibits the tumorigenesis and progression of breast cancer." CANCER RESEARCH 15 JUL 2005, vol. 65, no. 14, 15 July 2005 (2005-07-15), pages 6139-6150, XP002562136 ISSN: 0008-5472
- CHAO ET AL.: 'Natural antisense mRNAs to Hyaluronan biosynthesis and cell proliferation' J. BIOL. CHEM. vol. 280, no. 30, 2005, pages 27513 - 27522, XP008129411
- UDABAGE ET AL.: 'Antisense-mediated suppression of hyaluronan synthase 2 inhibits the tumorigenesis and progression of breast cancer' CANCER RESEARCH vol. 65, no. 14, 2005, pages 6139 - 6150, XP002562136
- UDABAGE ET AL.: 'The over-expression o HAS2, Hyal-2 and CD44 is implicated in the invasiveness of breast cancer' EXPERIMENTAL CELL RESEARCH vol. 310, 2005, pages 205 - 217, XP005093320

## Description

### BACKGROUND OF THE INVENTION

### FIELD OF THE INVENTION

The present invention relates generally to the treatment and prophylaxis of cellular proliferation and in particular cancer ; More particularly, the present invention targets hyaluronan anabolism to facilitate control of cellular proliferative conditions such as cancer.

### DESCRIPTION OF THE PRIOR ART

Bibliographic details of the publications referred to in this specification are also collected at the end of the description.

Reference to any prior art in this specification is not, and should not be taken as, an acknowledgment or any form of suggestion that this prior art forms part of the general knowledge in any country.

Hyaluronan (HA) metabolism is an intricate balance between the rate of HA synthesis and degradation where depending upon the physiological role being played by the HA, the simultaneous synthesis and degradation is carefully controlled. Hyaluronan is synthesized by a family of distinct yet related transmembrane proteins termed hyaluronan synthase (HAS) isoforms HAS1, 2 and 3, which can be distinguished from one another with respect to temporal and differential expression during mouse embryogenesis and in mature tissues, respectively and also in the molecular weight of the HA produced. The extracellular matrix polysaccharide HA or its acidic form, hyaluronic acid, is a linear, high molecular weight polymer comprised of repeating disaccharide units of (β1-3) D-glucuronate-(β1-4)N-acetyl-D-glucosamine (Weissman & Meyer, J. Am. Cham. Soc. 76:1753, 1954).

The extracellular matrix (ECM) is one of the crucial environmental elements that affect tumor cell behaviour. ECM serves as a scaffold to which tumor cells adhere and migrate as well as acting as a reservoir of growth factors and cytokines that are potentially beneficial to malignant cells (Ruoslahti & Yamaguchi, Cell 64:867-869, 1991). Hyaluronan (HA) is a negatively charged high molecular weight polysaccharide, which is an essential component of the extracellular matrix. For many years HA was considered the "space filler" of the ECM, but with the discovery HA cell surface receptors it has become apparent that it plays a more complex role in cell behaviour (Laurent & Fraser, FASEB J 6:2397-2404, 1992). More recently, HA has been associated with many different cellular hyperproliferative processes including cell division and migration as occurs during development (Toole, J. Internal Medicine 242:35-40, 1981), tissue remodelling (Knudson & Knudson, FASEB J. 7:1233, 1993), inflammation and tumor initiation, progression or invasion (Knudson et al, The Biology of Hyaluronan 143:150-169, 1989).

HAS1, the least active of the three HAS proteins, drives the synthesis of high molecular weight HA (2x10⁶D), thus HAS1 may play a role in maintaining a low, yet necessary level of HA synthesis in many cell types. HAS2 is widely expressed throughout embryonic development, where it is more active than HAS1 and it also synthesises high molecular weight HA (2x10⁶D) that is attached to the plasma membrane in the form of a pericellular gel (Fulop, Arch. Iochem. Iophys. 337:261-266, 1997). The HAS2 production of large amounts of high molecular weight HA may have a significant effect on tissue structure and volume, thus playing an important role in developmental processes involving tumor growth. HAS3 is the most active of the 3 HAS proteins, but drives the synthesis of short (<2x10⁵ to 3x10⁵D) HA chains. HAS3 expression may be activated to produce large amounts of low molecular weight HA, which may interact with cell surface HA receptors, triggering signalling cascades leading to changes in cell behaviour.

HAS2 is important in a highly invasive breast cancer cell line (Udabage et al, Cancer Res 65:6139-6150, 2005). Inhibition of HAS2 using antisense technology resulted in a 97% inhibition of cell invasiveness. Moreover, when antisense HAS2 cancer cells were implanted into nude mice, tumors did not initiate highlighting the potential therapeutic value of inhibiting a functional hyaluronan synthase in the malignant state.

These aforementioned studies highlight the importance of HAS isoforms in relation to cancer where HA synthesis can promote anchorage-independent growth and increase tumor invasiveness, properties that are hallmarks of the malignant phenotype.

The applicant has surprisingly discovered compounds, for example, antibodies, that interact with different sections of HAS amino acid sequence and which specifically target a binding epitope contained within the HAS epitope that is more accessible to an extracellular environment in disease associated cells compared to normal cells.

### SUMMARY OF THE INVENTION

The present invention is directed to compounds, agents, pharmaceutically active agents, medicaments, therapeutics, actives, drugs and the like which specifically target a portion of the HAS molecule which is accessible to the extracellular environment in a first form of a cell but which is not accessible to the extracellular environment in another form of or in a transformed cell form the same or related cell. In particular, the present invention provides compounds which target a portion of HAS which is accessible to the extracellular environment in malignant or proliferative cells but which portion is not accessible to the external environment in "normal" cells. A "normal" cell in this instance is a non-malignant, or proliferative cell.

The compounds are antibodies such as monoclonal or polyclonal antibodies. Pharmaceutical and other compositions comprising the compounds of the subject invention are also provided. Methods of screening for modulators of HAS activity in cells, tissues or animals are also contemplated including diagnostic assays for malignant, inflammatory and proliferative cells and, hence, conditions involving same. Methods of treating an animal, particularly a human, suspected of having or being prone to a disease or condition associated with HA levels or HAS activity are also set forth herein. Such methods comprise administering a therapeutically or prophylactically effective amount of one or more of the compounds or compositions of the present invention to the subject of need of treatment or suspected of needing treatment or prophylaxis.

The present invention provides, therefore, in one embodiment, compounds which selectively reduce HAS activity in selected cell types but which substantially do not reduce HAS activity in non-selected cell types. In particular, the present invention is directed to compounds which selectively target a portion of the HAS molecule which is accessible to the extracellular environment in a malignant, or proliferative cell but which portion is substantially not accessible to the extracellular environment in non-disease-associated cells.

In one aspect, the invention provides an isolated compound for reducing or altering the level of hyaluronan synthase (HAS) activity or the extracellular release of HA, wherein said compound targets an epitope of the HAS amino acid sequence wherein said epitope is more accessible to an extracellular environment in diseased cells compared to normal cells.

In another aspect, the invention provides an isolated compound capable of reducing or altering the level of hyaluronan synthase (HAS) activity or the extracellular release of HA, wherein said compound targets an epitope of the HAS amino acid sequence wherein said epitope is more accessible to an extracellular environment in diseased cells compared to normal cells.

The epitope is presented in the diseased cells in the extracellular space and in non-diseased cells in the intracellular space or plasma membrane.

The compound is a therapeutic antibody.

Said compound specifically targets an amino acid sequence INT-2 within an HAS or a sequence containing one or more conservative amino acid substitutions of INT-2.

Preferably, said compound specifically targets the amino acid sequence INT-2.

In another aspect, the invention provides a therapeutic antibody for use in a method of treatment of malignant or diseased cells comprising administering an a therapeutic antibody wherein such therapeutic antibody reduces or alters the level of hyaluronan synthase (HAS) activity, or extracellular release of HA, and said therapeutic antibody targets an epitope of the HAS amino acid sequence wherein said sequence is more accessible to an extracellular environment in disease associated cells compared to normal cells.

The epitope is present in the diseased cells in the extracellular space and in non-diseased cells in the intracellular space or plasma membrane.

The therapeutic antibody reduces the level of hyaluronan synthase (HAS) activity wherein said therapeutic antibody targets an amino acid sequence INT-2 within an HAS or a sequence containing one or more conservative amino acid substitutions of INT-2

Preferably, the therapeutic antibody specifically targets an amino acid sequence INT-2.

Preferably, the malignant or diseased cells are breast cancer cells.

Preferably, the hyaluronan synthase is selected from the group consisting of isoforms HAS I, HAS II and HAS III.

Preferably, the isoform is HAS II.

In another aspect, the invention provides use of a therapeutic antibody in the preparation of a medicament for the treatment of malignant or diseased cells wherein said compound reduces or alters the level of hyaluronan synthase (HAS) activity, or the extracellular release of HA, and targets an epitope contained within the HAS amino acid sequence wherein said epitope is more accessible to an extracellular environment in disease associated cells compared to normal cells

In another aspect, the invention provides a therapeutic antibody, capable of reducing the level of hyaluronan synthase (HAS) activity, or extracellular release of HA, wherein said therapeutic antibody, targets an epitope of the HAS amino acid sequence wherein said epitope is more accessible to an extracellular environment in disease associated cells compared to normal cells. Preferably, the therapeutic antibody, specifically targets HAS epitope INT-2.

**Table 1:**

| ***Sequence Identifiers*** | |
|---|---|
| **Sequence Identifier** | **Sequence** |
| 1 | Immunizing peptide - HAS418 (INT-1) |
| 2 | Immunizing peptide - HAS 419 (EX-1) |
| 3 | Immunizing peptide - HAS421 (INT-2) |
| 4 | Protein Human Enzyme |
| 5 | Protein Human Enzyme |
| 6 | Protein Human Enzyme |

### BRIEF DESCRIPTION OF THE FIGURES

**Figure 1** is a graphical representation of multiple amino acid alignment between the human hyaluronan synthase (HAS) family.
**Figure 2** is a graphical representation of the predicted topology in the non-malignant state
**Figure 3** is a photographic representation showing non-reduced conditions a single band outside the range of the molecular weight standards was detected. This band is estimated to be greater than 210,000Da and may represent accessory proteins required to drive the supply of HA precursors, notably UDP-glucuronate and UDP-N-acetylglucosamine requires for HA synthesis.
**Figure 4** is a graphical representation showing the growth rate of dermal fibroblasts unaffected in the presence of either INT-1 or INT-2 or EX-1.
**Figure 5** is a graphical representation showing a result of typical histogram plots obtained when dermal fibroblasts are reacted with sheep IgG.
**Figure 6A through C** are graphical representations showing the flow cytometric analysis of the HAS antibodies on dermal fibroblasts. Dermal fibroblasts were reacted with a) INT-1; b) INT-2; and c) EX-1. The filled (grey) plots represent the HAS antibody and the unfilled plot represents background fluorescence generated by corresponding control antibody. Note the shift in fluorescence intensity in panel c indicating a reactive epitope present at the cell surface.
**Figure 7** is a graphical representation showing the effect of HAS antibodies on cellular proliferation.
**Figure 8** is a graphical representation showing the inhibition of HA production by application of the HAS antibodies in malignant breast cancer cells.
**Figure 9** is a photographic representation of the effect of HAS antisera on cellular morphology. Cell line shown; MDA-MB-231. Magnification of each micrograph is 400X.
**Figure 10A and B** are photographic representations of DAPI staining. (A) MDA-MB-231 (B) MDA-MD-468. Note: no nuclear fragmentation detected.
**Figure 11A and B** are photographic representations of: (A) DNA extracted cell pellet after incubation of breast cancer with HAS antisera. No DNA fragment detected. (B) DNA extracted from cell lysate representing the cytosolic fraction. No fragmented DNA was detected in the cytosol.

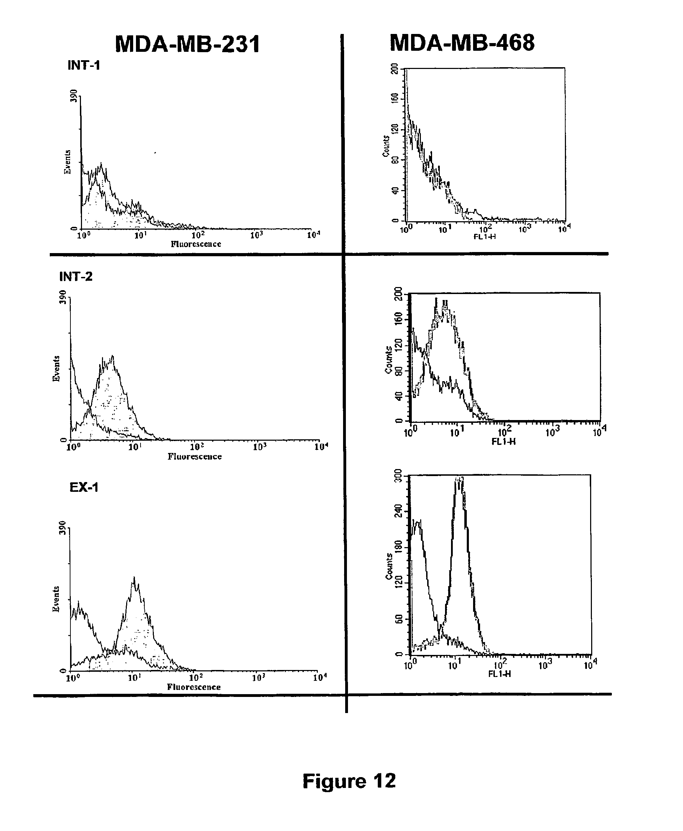

**Figure 12** is a graphical representation of MDA-MB-231 and MDA-MB-468.

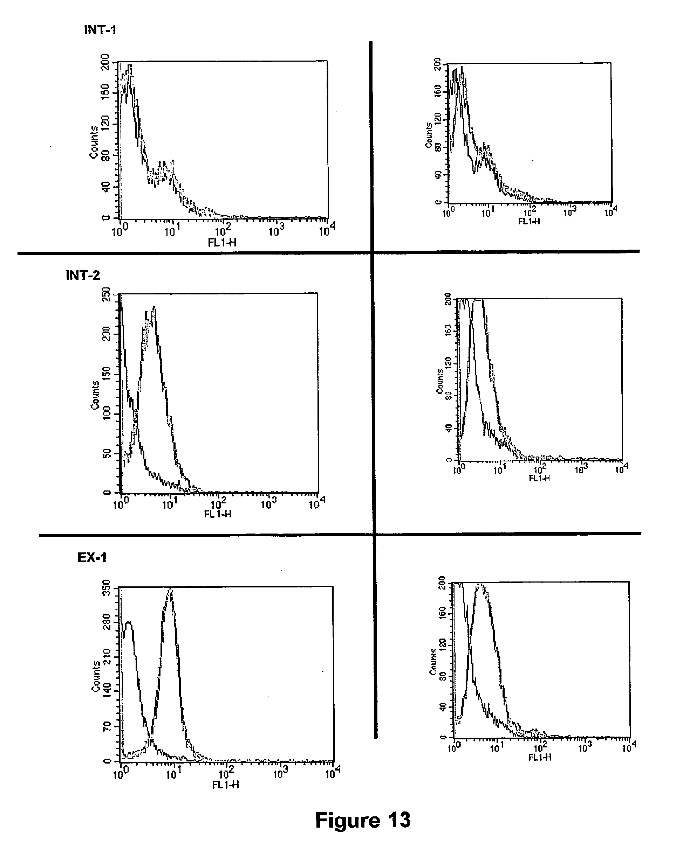

**Figure 13** is a graphical representation of ZRL-75-1 and MDA-MB-435. **Note:** Where histograms are presented with grey filled plots this represents the reactivity to the HAS antibody and the unfilled plot the corresponding antibody control. Where plots are presented in black lines these represent the background fluorescence as measured from the secondary-FITC only control. The green plots represent the reactivity of each HAS antibody to the cited breast cancer cell lines. The FACS data derived from the 4 breast cancer cell lines tested strongly suggest that in the malignant state the epitope for EX-1 and INT-2 are expressed extracellularly.

### DETAILED DESCRIPTION OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

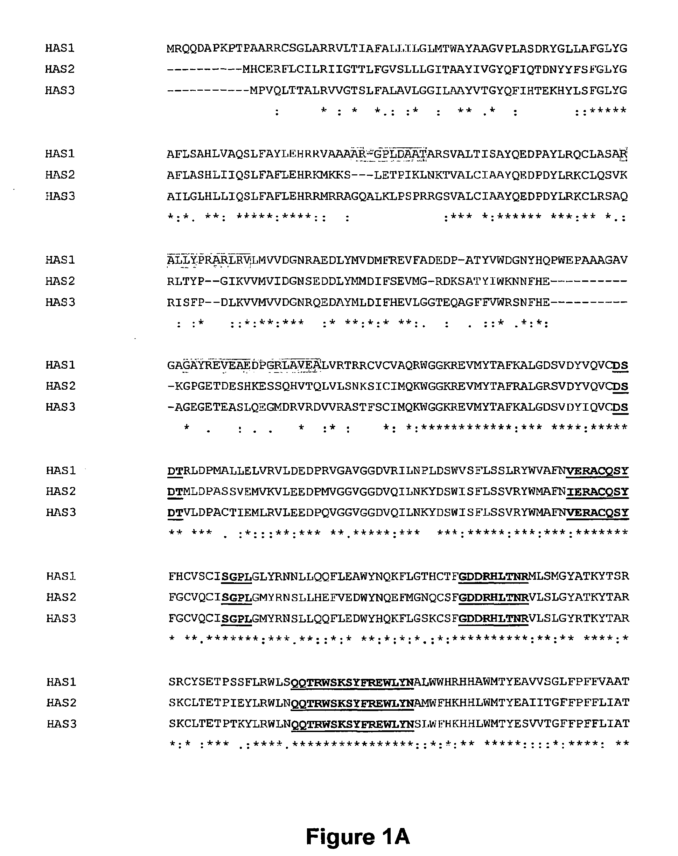

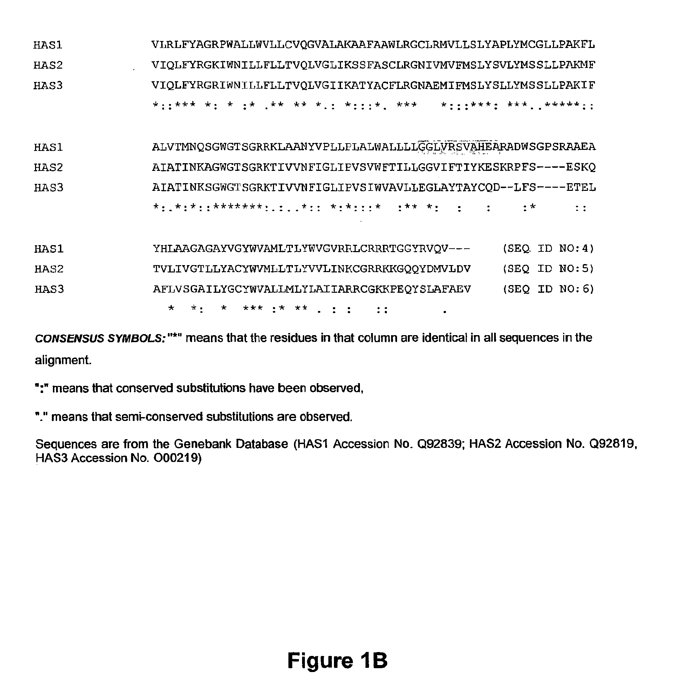

Examination of proposed intracellular loops of HAS revealed the presence of the most significant homology and conserved motifs (Figure 1; bolded/underlined amino acids). The HAS protein family is shown in Figure 1 where the protein sequences have been aligned. HAS1, 2 and 3 are 578, 552 and 553 amino acids in length, respectively with predicted molecular weights ranging from 63.1 to 64.8 *M*ᵣ (x 10³). At the amino acid level all members display approximately 52 to 85% identity. Based on amino acid substitution studies it has been shown that key motifs, shown in bold, are essential for HAS activity. The leucine residue in the SGPL motif (bolded) when substituted with a valine causes HAS activity to be lost.

Hence, the present invention identifies that in cancer cells INT-2 portions of HAS are accessible to an extracellular environment. In "normal" cells, i.e. cells not associated with cancer, these INT-2 are not accessible to the extracellular environment. The topology of the HAS molecule changes, therefor, depending on the state of the cell enabling new targets to generate compounds which selectively target HAS in disease-associated cells.

Hence, one aspect of the present invention is directed to a compound which selectively target an INT-2 portion of the HAS molecule which is accessible to the extracellular environment in a malignant, or proliferative cell but which INT-2 portion is substantially not accessible to the extracellular environment in non-disease-associated cells.

The terms "compound", "agent", "reagent", "pharmacologically active agent", "medicament", "therapeutic", "active" and "drug" are used interchangeably herein to refer to an antibody or other interactive molecule which is capable of binding to an INT-2 portion of HAS which is accessible to the extracellular environment in a malignant, or proliferative cell but which is substantially not accessible in a normal cell. The terms also encompass pharmaceutically acceptable and pharmacologically active ingredients of those active agents specifically mentioned herein. When the terms "agent", "reagent", "compound", "pharmacologically active agent", "medicament", "therapeutic", active" and "drug" are used, then it is to be understood that this includes the active entity *per se* as well as pharmaceutically acceptable, pharmacologically active salts, chimeras and recombinant forms of the antibodies.

Reference to an "agent", "chemical agent", "compound", "pharmacologically active agent", "medicament", "therapeutic", "active" and "drug" includes combinations of two or more active agents. A "combination" also includes multi-part such as a two-part composition where the agents are provided separately and given or dispensed separately or admixed together prior to dispensation. For example, a multi-part pharmaceutical pack may have two or more agents separately maintained. Hence, this aspect of the present invention includes combination therapy. Combination therapy includes the co-administration of two antibodies, each specific for a portion of HAS which is only accessible to the external environment in disease-associated cells.

The terms "effective amount" and "therapeutically effective amount" of an agent as used herein mean a sufficient amount of the agent to provide the desired therapeutic or physiological or effect or outcome. Such an effect or outcome includes reduction or amelioration of the symptoms of cellular disease. Undesirable effects, e.g. side effects, are sometimes manifested along with the desired therapeutic effect; hence, a practitioner balances the potential benefits against the potential risks in determining what is an appropriate "effective amount". The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation. Generally, the agent or agents is/are given in an amount and under conditions sufficient to reduce proliferation of cells including malignant cells.

The present invention employs antibodies directed to the subject HAS molecule for use in modulating the function of HAS and, in a particular embodiment, HAS1, HAS2 and/or HAS3. As used herein, reference to HAS includes any molecule with the same function. In a preferred aspect, reference to "HAS", includes reference to the isoforms HAS1, HAS2 or HAS3. The antibodies are specific for portions of the HAS exposed or accessible to the extracellular environment in disease-associated cells.

Accordingly, the present invention provides an isolated antibody capable of selectively affecting the function or activity of HAS in diseased versus non-diseased cells.

The present invention provides, therefore, antagonists of HAS function or activity. Such antagonists are useful in reducing the effects of HAS and hence reducing or elevating levels of HAS.

Accordingly, in a preferred aspect the present invention provides antibodies that bind, interact or otherwise associate with HAS and which reduce HAS function or activity in disease-associated cells but not to normal cells.

As indicated above, a "normal" cell is a cell not associated with a malignancy, or proliferation.

The antibodies maybe monoclonal or polyclonal antibodies, although, monoclonal antibodies are preferred. Generally, the antibodies are in isolated, homogenous or fully or partially purified form.

The antibodies may also be humanized or chimeric or are human antibodies suitable for administration to humans. These include humanized antibodies prepared, for example, from murine monoclonal antibodies, and human monoclonal antibodies which may be prepared, for example, using transgenic mice as described below, or by phage display. A "humanized" antibody includes a deimmunized antibody.

Antibodies are raised against a HAS such as HAS 1, 2 or 3 or immunogenic parts thereof or immunologically homologous molecules. Antibodies are raised against HAS epitopes (INT-2) that are differentially exposed to an environment in diseased versus non-diseased cells.

Reference to an "antibody" or "antibodies" includes reference to all the various forms of antibodies, including but not limited to: full antibodies (e.g. having an intact Fc region), including, for example, monoclonal antibodies; antigen-binding antibody fragments, including, for example, Fv, Fab, Fab' and F(ab')₂ fragments; humanized antibodies; human antibodies (e.g., produced in transgenic animals or through phage display); and immunoglobulin-derived polypeptides produced through genetic engineering techniques. Unless otherwise specified, the terms "antibody" or "antibodies" and as used herein encompasses both full antibodies and antigen-binding fragments thereof.

Unless stated otherwise, specificity in respect of an antibody of the present invention is intended to mean that the antibody binds substantially only to its target antigen with no appreciable binding to unrelated proteins. However, it is possible that an antibody will be designed or selected to bind to two or more related proteins. A related protein includes different splice variants or fragments of the same protein or homologous proteins from different species. Such antibodies are still considered to have specificity for those proteins and are encompassed by the present invention. The term "substantially" means in this context that there is no detectable binding to a non-target antigen above basal, i.e. nonspecific, levels.

The antibodies of the present invention may be prepared by well known procedures. See, for example, Monoclonal Antibodies, Hybridomas: A New Dimension in Biological Analyses, Kennet et al, (eds.), Plenum Press, New York (1980); and Antibodies: A Laboratory Manual, Harlow and Lane (eds.), Cold Spring Harbor Laboratory Press, Cold Spring Harbor, NY, (1988).

The present invention contemplates a method for producing a hybridoma cell line which comprises immunizing a non-human animal, such as a mouse or a transgenic mouse, with a HAS or immunogenic parts thereof; harvesting spleen cells from the immunized animal; fusing the harvested spleen cells to a myeloma cell line to generate hybridoma cells; and identifying a hybridoma cell line that produces a monoclonal antibody that binds to a HAS.

Such hybridoma cell lines and the HAS monoclonal antibodies produced by them are encompassed by the present invention. Monoclonal antibodies secreted by the hybridoma cell lines are purified by conventional techniques. Hybridomas or the monoclonal antibodies produced by them may be screened further to identify monoclonal antibodies with particularly desirable properties.

The HAS molecule or immunogenic part thereof that may be used to immunize animals in the initial stages of the production of the antibodies of the present invention may be from any mammalian source.

Antigen-binding fragments of antibodies of the present invention may be produced by conventional techniques. Examples of such fragments include, but are not limited to, Fab, Fab', F(ab')₂ and Fv fragments, including single chain Fv fragments (termed sFv or scFv). Antibody fragments and derivatives produced by genetic engineering techniques, such as disulphide stabilized Fv fragments (dsFv), single chain variable region domain (Abs) molecules, minibodies and diabodies are also contemplated for use in accordance with the present invention.

Such fragments and derivatives of monoclonal antibodies directed against HAS molecules may be prepared and screened for desired properties, by known techniques, including the assays described herein. Certain of the techniques involve isolating DNA encoding a polypeptide chain (or a portion thereof) of a mAb of interest, and manipulating the DNA through recombinant DNA technology. The DNA may be fused to another DNA of interest, or altered (e.g. by mutagenesis or other conventional techniques) to add, delete, or substitute one or more amino acid residues, for example.

DNA encoding antibody polypeptides (e.g. heavy or light chain, variable region only or full length) may be isolated from B-cells of mice that have been immunized with modified LIF molecules. The DNA may be isolated using conventional procedures. Phage display is another example of a known technique whereby derivatives of antibodies may be prepared. In one approach, polypeptides that are components of an antibody of interest are expressed in any suitable recombinant expression system, and the expressed polypeptides are allowed to assemble to form antibody molecules.

Single chain antibodies may be formed by linking heavy and light chain variable region (Fv region) fragments via an amino acid bridge (short peptide linker), resulting in a single polypeptide chain. Such single-chain Fvs (scFvs) have been prepared by fusing DNA encoding a peptide linker between DNAs encoding the two variable region polypeptides (VL and VH). Single chain antibodies derived from antibodies provided herein are encompassed by the present invention.

In one embodiment, the present invention provides antibody fragments or chimeric, recombinant or synthetic forms of the antibodies of the present invention that bind to a HAS such as HAS1, 2 and/or 3.

Techniques are known for deriving an antibody of a different subclass or isotype from an antibody of interest, i.e., subclass switching. Thus, IgG1 or IgG4 monoclonal antibodies may be derived from an IgM monoclonal antibody, for example, and vice versa. Such techniques allow the preparation of new antibodies that possess the antigen-binding properties of a given antibody (the parent antibody), but also exhibit biological properties associated with an antibody isotype or subclass different from that of the parent antibody. Recombinant DNA techniques may be employed. Cloned DNA encoding particular antibody polypeptides may be employed in such procedures, e.g. DNA encoding the constant region of an antibody of the desired isotype.

The monoclonal production process described above may be used in animals, for example mice, to produce monoclonal antibodies. Conventional antibodies derived from such animals, for example murine antibodies, are known to be generally unsuitable for administration to humans as they may cause an immune response. Therefore, such antibodies may need to be modified in order to provide antibodies suitable for administration to humans. Processes for preparing chimeric and/or humanized antibodies are well known in the art and are described in further detail below.

The monoclonal antibodies herein specifically include "chimeric" antibodies in which the variable domain of the heavy and/or light chain is identical with or homologous to corresponding sequences in antibodies derived from a non-human species (e.g., murine), while the remainder of the chain(s) is identical with or homologous to corresponding sequences in antibodies derived from humans, as well as fragments of such antibodies, so long as they exhibit the desired biological activity

"Humanized" forms of non-human (e.g., murine) antibodies are chimeric antibodies which contain minimal sequence derived from the non-human immunoglobulin. For the most part, humanized antibodies are human immunoglobulins (recipient antibody) in which the complementarity determining regions (CDRs) of the recipient are replaced by the corresponding CDRs from a non-human species (donor antibody) such as mouse, rat, rabbit or nonhuman primate having the desired properties, for example specificity, and affinity. In some instances, framework region residues of the human immunoglobulin are replaced by corresponding non-human residues. Furthermore, humanized antibodies may comprise residues which are not found in the recipient antibody or in the donor antibody.

These modifications are made to further refine antibody performance. In general, the humanized antibody will comprise substantially all of at least one, and typically two, variable domains, in which all or substantially all of the complementarity determining regions correspond to those of a non-human immunoglobulin and all or substantially all of the framework region residues are those of a human immunoglobulin sequence. The humanized antibody optionally also will comprise at least a portion of an immunoglobulin constant region (Fc), typically that of a human immunoglobulin.

Procedures for generating human antibodies in non-human animals have been developed and are well known to those skilled in the art. For example, transgenic mice into which genetic material encoding one or more human immunoglobulin chains has been introduced may be used to produce the antibodies of the present invention. Antibodies produced in the animals incorporate human immunoglobulin polypeptide chains encoded by the human genetic material introduced into the animal.

Another method for generating human antibodies is phage display. Phage display techniques for generating human antibodies are well known to those skilled in the art, and include the methods used by companies such as Cambridge Antibody Technology and MorphoSys.

The antibodies described herein can also be applied in the areas of drug discovery and target validation. This includes the use of the antibodies and preferred target segments identified herein in drug discovery efforts to elucidate relationships that exist between HA, HAS or HA/HAS interaction and a disease state, phenotype, or condition. These methods include detecting or modulating HAS comprising contacting a sample, tissue, cell, or organism with the compounds of the present invention, measuring the nucleic acid or protein level of HAS and/or a related phenotypic or chemical endpoint at some time after treatment, and optionally comparing the measured value to a non-treated sample or sample treated with a further compound of the invention. These methods can also be performed in parallel or in combination with other experiments to determine the function of unknown genes for the process of target validation or to determine the validity of a particular gene product as a target for treatment or prevention of a particular disease, condition, or phenotype.

The present invention contemplates the use of the antibodies described herein as therapeutic agents to treat subjects suffering from cancer associated with HA. Subjects treated using the compositions and antibodies of the present invention include any animal that may benefit from such treatment. These include, without limitation, humans, marmosets, orangutans and gorillas, livestock animals (e.g. cows, sheep, pigs, horses, donkeys), laboratory test animals (e.g. mice, rats, guinea pigs, hamsters, rabbits), companion animals (e.g. cats, dogs) and captured wild animals (e.g. rodents, foxes, deer, kangaroos. A particularly preferred host is a human, primate or livestock animal.

The antibodies of the present invention can be utilized for diagnostics, therapeutics, prophylaxis and as research reagents and kits. Furthermore, antibodies to HAS which are able to affect HAS and with exquisite specificity, are often used by those of ordinary skill to elucidate the function of particular genes or gene products or to distinguish between functions of various members of a biological pathway.

For use in kits and diagnostics, the antibodies of the present invention, either alone or in combination with other compounds or therapeutics, can be used as tools in differential and/or combinatorial analyses to elucidate expression patterns of a portion or the entire complement of genes expressed within cells and tissues.

The antibodies of the invention are useful for research and diagnostics, because these antibodies bind to HAS itself. For example, antibodies may be labeled with reporter molecules including enzymes and radiolabels for imaging purposes, diagnostic purposes or quantitative purposes. Kits using such detection means for detecting the level of HAS in a sample may also be prepared.

The specificity and sensitivity of antibodies are also harnessed by those of skill in the art for therapeutic uses. Such compounds have been employed as therapeutic moieties in the treatment of disease states in animals, including humans.

For therapeutics, an animal, preferably a human, suspected of having a disease or disorder which can be treated by modulating the expression of the HAS can be treated with antibodies to inhibit HAS activity. For example, in one non-limiting embodiment, the methods comprise the step of administering to the animal in need of treatment, a therapeutically effective amount of an antibody which selectively inhibits HAS expression in diseased cells. In one embodiment, the activity or expression of HAS in an animal is inhibited by about 10%. Preferably, the activity or expression of HAS in an animal is inhibited by about 30%. More preferably, the activity or expression of HAS in an animal is inhibited by 50% or more.

For example, the reduction of the expression of the HAS gene may be measured in serum, adipose tissue, skin cells, liver or any other body fluid, tissue or organ of the animal. Preferably, the cells contained within said fluids, tissues or organs being analyzed contain a nucleic acid molecule encoding a HAS protein.

The present invention contemplates, therefore, methods of screening for compounds comprising, for example, contacting a candidate compound with HAS. The screening procedure includes assaying (i) for the presence of a complex between the drug and HAS and (ii) screening for an alteration in the expression levels of HAS in diseased and non-diseased cells. Whole cells may also be screened for interaction between the cell and the drug.

One form of assay involves competitive binding assays. In such competitive binding assays, the candidate compound or HAS is typically labeled. Free HAS is separated from any putative complex and the amount of free (i.e. uncomplexed) label is a measure of the binding of the agent being tested to target molecule. One may also measure the amount of bound, rather than free, HAS. It is also possible to label the compound rather than HAS and to measure the amount of compound binding HAS in the presence and in the absence of the compound being tested. Such compounds may inhibit HAS which is useful, for example, in finding HAS inhibitors.

Another technique for drug screening provides high throughput screening for compounds having suitable binding affinity to a target Briefly stated, large numbers of different small peptide test compounds are synthesized on a solid substrate, such as plastic pins or some other surface. The peptide test compounds are reacted with HAS and washed. Bound HAS molecules are then detected by methods well known in the art. This method may be adapted for screening for non-peptide, chemical entities. This aspect, therefore, extends to combinatorial approaches to screening for HAS antagonists or agonists.

Purified HAS can be coated directly onto plates for use in the aforementioned drug screening techniques. However, non-neutralizing antibodies to the target may also be used to immobilize the target on the solid phase.

It must be noted that, as used in the subject specification, the singular forms "a", "an" and "the" include plural aspects unless the context clearly dictates otherwise. Thus, for example, reference to "a compound" includes a single compound, as well as two or more compounds; reference to "an antibody" includes a single antibody, as well as two or more antibodies; and so forth.

The terms "compound", "active agent", "pharmacologically active agent", "medicament", "active" and "drug" are used interchangeably herein to refer to an antagonist of HAS function or activity or of expression of genetic material encoding same which induces a desired pharmacological and/or physiological effect such as but not limited to controlling inflammation and reducing cancer growth. The terms also encompass pharmaceutically acceptable and pharmacologically active ingredients of those active agents specifically mentioned herein including but not limited to salts, esters, amides, prodrugs, active metabolites, analogs and the like. When the terms "compound", "active agent", "pharmacologically active agent", "medicament", "active" and "drug" are used, then it is to be understood that this includes the active agent *per se* as well as pharmaceutically acceptable, pharmacologically active salts, esters, amides, prodrugs, metabolites, analogs, etc.

The terms "effective amount" and "therapeutically effective amount" of the compound as used herein mean a sufficient amount of the agent to provide the desired therapeutic or physiological effect such as inhibiting inflammation or reducing the growth or spread of cancer cells. Undesirable effects, e.g. side effects, are sometimes manifested along with the desired therapeutic effect; hence, a practitioner balances the potential benefits against the potential risks in determining what is an appropriate "effective amount". The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation. The present invention extends to a method of treatment or prophylaxis.

By "pharmaceutically acceptable" carrier, excipient or diluent is meant a pharmaceutical vehicle comprised of a material that is not biologically or otherwise undesirable, i.e. the material may be administered to a subject along with the selected active agent without causing any or a substantial adverse reaction. Carriers may include excipients and other additives such as diluents, detergents, coloring agents, wetting or emulsifying agents, pH buffering agents, preservatives, and the like.

Similarly, a "pharmacologically acceptable" salt, ester, amide, prodrug or derivative of a compound as provided herein is a salt, ester, amide, prodrug or derivative that this not biologically or otherwise undesirable.

The terms "treating" and "treatment" as used herein refer to reduction in severity and/or frequency of symptoms of diseases or disorders or physiological conditions elimination of symptoms and/or underlying cause, prevention of the occurrence of symptoms of disease and/or their underlying cause and improvement or remediation of conditions associated with cytokine activity.

"Treating" a patient may involve prevention of the disorder or disease condition or physiological event in a susceptible individual as well as treatment of a clinically symptomatic individual by inhibiting a disease or disorder.

Accordingly, another aspect of the present invention is directed towards therapeutic or prophylactic composition comprising an antibody capable of reducing the levels or activity of HAS hence reducing levels of HA.

The compositions and antibodies of the present invention can be used in the treatment or prevention of cancer associated with HA.

The antibodies of the invention can be utilized in pharmaceutical compositions by adding an effective amount of an antibody to a suitable pharmaceutically acceptable diluent or carrier. Use of the antibodies and methods of the invention may also be useful prophylactically.

The antibodies of the invention may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption.

The term "prodrug" indicates a therapeutic agent that is prepared in an inactive form that is converted to an active form (i.e., drug) within the body or cells thereof by the action of endogenous enzymes or other chemicals and/or conditions. I

The term "pharmaceutically acceptable salts" refers to physiologically and pharmaceutically acceptable salts of the compounds of the invention: i.e., salts that retain the desired biological activity of the parent compound and do not impart undesired toxicological effects thereto.

The present invention also includes pharmaceutical compositions and formulations which include the interactive antibodies of the present invention. The pharmaceutical compositions of the present invention may be administered in any number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration. The antibodies may be modified for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, gel capsules, liquid syrups, soft gels, suppositories, and enemas. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

Pharmaceutical compositions of the present invention include, but are not limited to, solutions, emulsions, foams and liposome-containing formulations. The pharmaceutical compositions and formulations of the present invention may comprise one or more penetration enhancers, carriers, excipients or other active or inactive ingredients.

Emulsions are typically heterogenous systems of one liquid dispersed in another in the form of droplets usually exceeding 0.1 µm in diameter. Emulsions may contain additional components in addition to the dispersed phases, and the active drug which may be present as a solution in either the aqueous phase, oily phase or itself as a separate phase. Microemulsions are included as an embodiment of the present invention.

Formulations of the present invention include liposomal formulations. As used in the present invention, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered.

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety.

The pharmaceutical formulations and compositions of the present invention may also include surfactants. The use of surfactants in drug products, formulations and in emulsions is well known in the art.

One of skill in the art will recognize that formulations are routinely designed according to their intended use, i.e. route of administration.

Preferred formulations for topical administration include those in which the antibodies of the invention are in admixture with a topical delivery agent such as lipids, liposomes, fatty acids, fatty acid esters, steroids, chelating agents and surfactants. Preferred lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA).

For topical or other administration, antibodies of the invention may be encapsulated within liposomes or may form complexes thereto, in particular to cationic liposomes. Alternatively, antibodies may be complexed to lipids, in particular to cationic lipids.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Preferred oral formulations are those in which oligonucleotides of the invention are administered in conjunction with one or more penetration enhancers surfactants and chelators. Preferred surfactants include fatty acids and/or esters or salts thereof, bile acids and/or salts thereof. Also preferred are combinations of penetration enhancers, for example, fatty acids/salts in combination with bile acids/salts. A particularly preferred combination is the sodium salt of lauric acid, capric acid and UDCA. Further penetration enhancers include polyoxyethylene-9-lauryl ether, polyoxyethylene-20-cetyl ether. Antibodies of the invention may be delivered orally, in granular form including sprayed dried particles, or complexed to form micro or nanoparticles.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Certain embodiments of the present invention provide pharmaceutical compositions containing one or more antibodies and one or more other chemotherapeutic agents. Examples of such chemotherapeutic agents include but are not limited to cancer chemotherapeutic drugs such as daunarubicin, daunomycin, dactinomycin, doxorubicin, epirubicin, idarubicin, esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5-azacytidine, hydroxyurea, deoxycoformycin, 4-hydroxyperoxycyclophosphoramide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin and diethylstilbestrol (DES). When used with the antibodies of the invention, such chemotherapeutic agents may be used individually (e.g., 5-FU and oligonucleotide), sequentially (e.g., 5-FU and oligonucleotide for a period of time followed by MTX and oligonucleotide), or in combination with one or more other such chemotherapeutic agents (e.g., 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. Two or more combined compounds may be used together or sequentially.

In another related embodiment, compositions of the invention may contain one or more antibodies targeted to a first protein and one or more additional compounds targeted to a second protein. Alternatively, compositions of the subject invention may contain two or more antibodies targeted to different regions of the same protein.

The formulation of therapeutic compositions and their subsequent administration (dosing) is within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual compounds, and can generally be estimated based on EC₅₀s found to be effective in *in vitro* and *in vivo* animal models. In general, dosage is from 0.01 ug to 100 g per kg of body weight, and may be given once or more daily, weekly, monthly or yearly, or even once every 2 to 20 years. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the antibody is administered in maintenance doses, ranging from 0.01 µg to 100 g per kg of body weight, once or more daily, to once every 20 years. Examples of effective amounts include 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100g/kg body weight.

Common to all HAS proteins are the hydrophobic sequences in their structures which would predict a plasma membrane association. Predicted clusters of the transmembrane domains are seen at either end of the protein which varies from 1-2 in number at the amino terminal and from 4-6 at the carboxyl terminus. The length of the cytosolic loop for the eukaryotic members varies from 307 to 328 residues and probably contains the catalytic domains to direct HA synthesis.

On the basis of the predicted amino acid sequence for HAS1, determined by Itano & Kimata, *Biochm. Biophys. Res. Comm. 222*:816-821, 1996, three short antigenic peptides were designed and synthesized by solid phase amino acid synthesis and purified by reverse-phase high-pressure chromatography. The peptides were determined to be 99.9% pure as shown by mass spectrometry. The production, purification, conjugation to diphtheria toxoid (DT), and purity testing of the peptides were performed by Chiron Mimitopes (Melbourne, Victoria, Australia). The sequence of each of the three immunizing peptides were designated HAS418 (INT-1), HAS419 (EX-1) and HAS421 (INT-2). Theses immunizing peptides were the subject of an earlier patent application by the inventors (PCT/AU04/01383; WO05/035548).

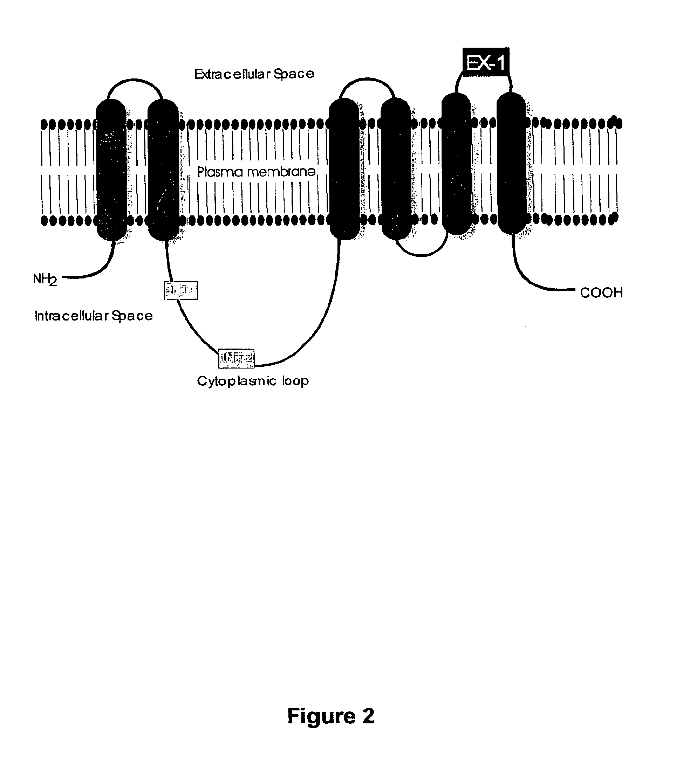

Using a transmembrane prediction program (TM predict) each synthase contains 6-7 putative transmembrane domains. In accord with preliminary data based upon the reactivity of the HAS antisera to non-malignant cells such as dermal fibroblasts a putative model of how the synthase is orientated in the plasma membrane can be proposed (see Figure 2). The EX-1 epitope is located between TMD 5 and 6. As TMD is the last hydrophobic sequence in the synthase this places the carboxyl terminus within the cytoplasm. The predicted topology of the hyaluronan synthase with locations of the epitopes for the HAS antisera is shown.

In work leading up to the present invention, it was observed that the antibodies to EX-1, INT-1 and INT-2 were able to affect the activity of the different HAS isoforms in normal and/or malignant cells; despite the fact that, based on the predicted amino acid sequence (Figure 1), these antibodies were expected to only affect activity of HAS 1. Furthermore, it was found that HA synthase topology of non-malignant cells appeared to be consistent with the predicted topology, however, surprisingly, in breast cancer cells altered topology was apparent. These data warranted further evaluation of inhibitory antibodies specific to novel HAS epitopes which could potentially act as selective therapeutic inhibitors of hyaluronan synthesis, thereby leading to new therapies for cancer. Hence, HAS, and in particular, HAS 1, 2 and/or 3 represent useful drug targets.

The present invention is further described by the following non-limiting Examples.

### EXAMPLE 1

### Production of HAS antiserum

On the basis of the predicted amino acid sequence for HAS 1 (Itano & Kimata, 1996), three short antigenic peptides were designed and synthesized by solid phase amino acid synthesis and purified by reverse-phase high-pressure chromatography. The peptides were determined to be 99.9% pure as shown by mass spectrometry. The production, purification, conjugation to diphtheria toxoid (DT), and purity testing of the peptides were performed by Chiron Mimitopes (Melbourne, Australia). The sequence of each Immunizing peptide is shown in Table 2.

**Table 2:**

| ***Immunizing peptide sequences and location*** | | | |
|---|---|---|---|
| ***Immunizing peptide*** | **Amino acid sequence** | **Putative location in topology of HAS** | |
| | | **Intracellular** | **Extracellular** |
| ***HAS418 (INT-1)*** | AARGPLDAATCRALLYPRARV (SEQ ID NO:1) | [ | |
| | **49→58 'Cys' 94→103** | | |
| ***HAS419 (EX-1)*** | GGLVRSVAHEA (SEQ ID NO:2) | | [ |
| | **480→490** | | |
| ***HAS421 (INT-2)*** | GAYREVEAEDPGRLAVE (SEQ ID NO:3) | [ | |
| | **146→162** | | |

| | | | |
|---|---|---|---|
| ***Note: please refer to amino acid alignment for locations of immunizing peptide sequences on HAS1 and their relationship between HAS2 and 3. These sequences do not align with any motifs that have been identified as essential for hyaluronan synthase activity*** | | | |

Border Leicester Merino cross-bred sheep were injected intramuscularly at two sites with the peptides (0.2-0.5mg) dissolved in complete Freund's adjuvant and again two weeks later in incomplete Freud's adjuvant. At day 35, the sheep were bled, and the serum separated by centrifugation, and stored at -20°C. All serum collected was tested with an enzyme-linked immunosorbent assay for antibodies specific for the peptide and carrier protein. The Immunizing peptide was coupled to thiopropyl-Sepharose 6B gel (Amersham Pharmacia Biotech, Uppsala, Sweden) by cyanogen bromide activation and the specific antibodies were extracted from the polyclonal sheep HAS antiserum by affinity chromatography. In brief 5mL of serum was mixed with 3mL of PBS and mixed with affinity/ligand resin for 1 hour at room temperature, followed by three washes of 5mL PBS. The antibodies were eluted in 0.1M glycine pH2.8 and were immediately neutralized to pH7.2 by the addition of 0.1M NaOH.

HAS polyclonal antibodies were then concentrated in an Amicon cell concentrator fitted with a YM30 Diaflo filter. The protein concentration of each affinity purified antibody was determined by the BCA assay (Pierce, U.S.A). The sterility of the antibodies used in immunohistochemistry or immunoblotting was assured by the addition of 0.1%w/v sodium azide, before storage at -20°C in aliquots. Antibodies intended for addition to cell cultures was stored at -20°C without azide.

### EXAMPLE 2

### Functional Characterization of the HAS antiserum

Initial crude plasma membrane preparations isolated from white blood cells were resolved in a 10% acrylamide gel under both reducing and non-reducing condition. Western blot anlaysis confirmed that under reducing conditions all three antibodies detected a single band of an approximate molecular mass of 60,000Da which is in accord with the predicted molecular weight of a hyaluronan synthase.

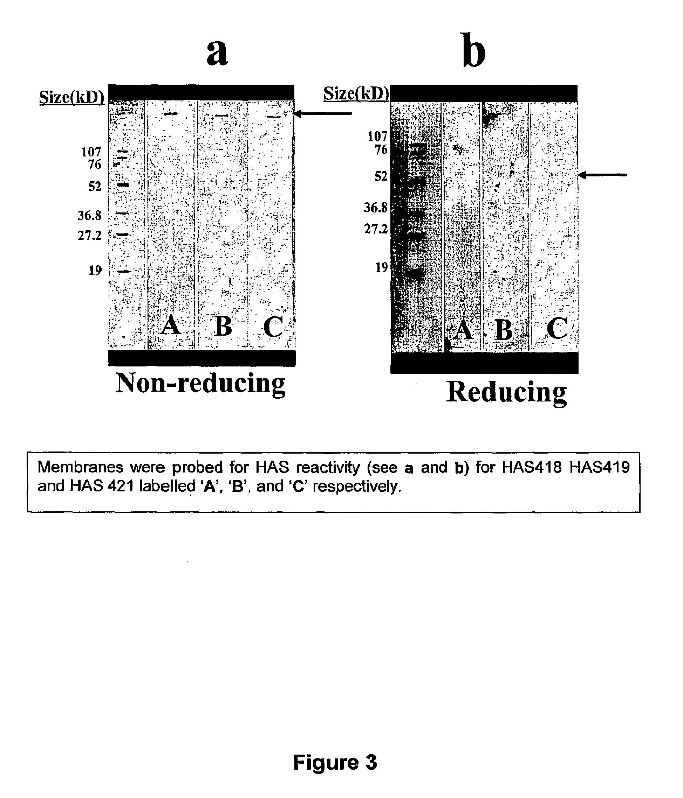

Under non-reduced conditions a single band outside the range of the molecular weight standards was detected. This band is estimated to be greater than 210,000Da and may represent accessory proteins required to drive the supply of HA precursors, notably UDP-glucuronate and UDP-N-acetylglucosamine requires for HA synthesis, refer Figure 3.

### EXAMPLE 3

### Effect of HAS antibodies on growth rate and hyaluronan synthesis in cultures of dermal fibroblasts

The effect of each hyaluronan synthase antibody on dermal fibroblasts in culture was examined. Monolayer cultures of three dermal fibroblast cell lines were established in 24-well tissue culture plates (17,000cells/mL medium/well). Cells were cultured in BME supplemented with 10% v/v FCS, 1.9mM glutamine, 20mM HEPES, 0.09% w/v bicarbonate and an antibiotic/antimycotic solution consisting of 100 units penicillin, 0.1mg streptomycin and 0.25µg/mL of amphotericin B at 37°C in 5% CO₂. After a settling period of 24 hours the mean plating efficiency and cell number were determined by trypsinising 4 wells in 0.25% (w/v) trypsin/EDTA in PBS and quantitating in a Model-ZM coulter counter. HAS antibodies 418, 419, 421 and sheep IgG were diluted in the culture medium to a final concentration of 300µg/mL and were applied to remaining wells, final volume per well was 1mL. Thereafter the medium was harvested and cell counts estimated at days 1, 2, 4, and 8. The culture medium was stored at 4°C with 0.02% sodium azide as preservative until HA was quantitated with the HA radiometric assay.

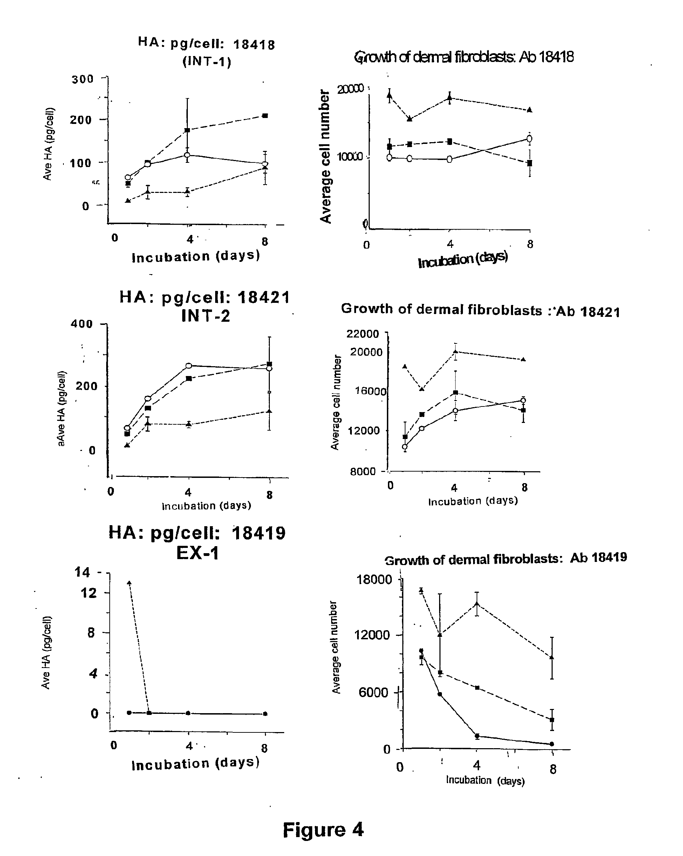

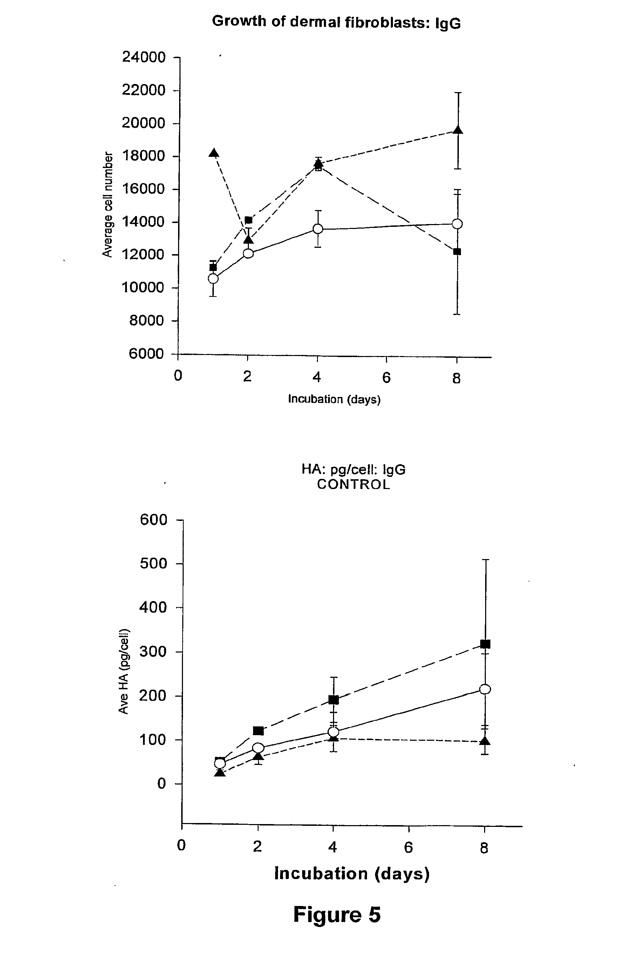

The growth rate of dermal fibroblasts was unaffected in the presence of either HAS418 (INT-1) or 421(INT-2) when compared with sheep IgG or no antibody controls. These results would be consistent with the presumption that the HAS418 and 421 epitopes are located intracellularly. The amount of HA synthesized in all experimental conditions was less in one of the three cell lines tested (Figure 4) which probably reflects the state of confluency of this particular cell line when examining the growth rate. The rate of hyaluronan synthesis is higher in actively proliferating cell cultures than in high density cultures with fewer cell divisions. The most striking result was the inhibitory effect of HAS419 (EX-1) on the synthesis of hyaluronan (Figure 4 and 5). Hyaluronan synthesis in cultures of dermal fibroblasts was completely inhibited after 24 hours incubation with 300µg/mL of HAS419. Cell numbers declined progressively over 1 or 2 to 8 days in all groups. This was correlated with a progressive drop in cell number. This would indicate that the epitope for HAS419 (EX-1) in dermal fibroblasts is located on the cell surface. The other hyaluronan synthase antibodies, HAS418 (INT-1) and 421 (INT-2) did not exert any HA synthesis inhibitory effect or affected the growth rate. The experimental system used for these experiments was complement-free to avoid any acute cell lysis resulting from antibody-antigen interaction with complement.

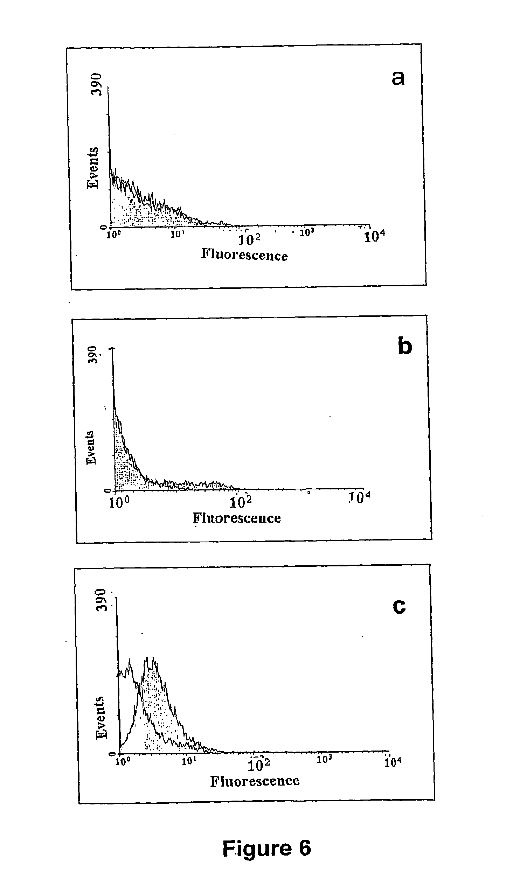

To confirm orientation of the HAS antibody epitopes further work was conducted utilising Fluorescence-activated Cell Sorter Analysis (FACS) on non-permeablised dermal fibroblasts. Figure 6a, 6b and 6c show a representative result of typical histogram plots obtained when dermal fibroblasts are reacted with INT-1,2 and EX-1, respectively.

The FACS results indicate that both epitopes, INT-1 and INT-2 are non-reactive in non-permeabalised dermal fibroblasts suggesting that these epitopes are located intracellularly. This correlates well with the functional studies performed where INT-1 and 2 antibodies did not perturb growth rate and hyaluronan synthesis. In contrast the epitope to EX-1 was shown to perturb HA synthesis and cause a reduction in cell number and reacted positively when analyzed by FACS. This indicates that the epitope for EX-1 is located on the cell surface.

When considering the transmembrane domains (TMD) of the hyaluronan synthase the epitope for EX-1 is found between TMD 5 and 6. This places the loop between these two TMDs on the outside of the cell. In addition as TMD 6 is the last hydrophobic segment it places the carboxyl terminus intracellulary.

### EXAMPLE 4

### Effect of HAS antibodies on growth rate and hyaluronan synthesis in cultures of breast cancer cell lines

Aneuploid human breast adenocarcinoma cell lines MDA-MB-468 and MDA-MB-231 were selected based on the differential expression of HA receptors, invasive/metastatic ability and extent of HA production.

Cell lines were plated into 24-well plates. Cells were allowed to adhere for 24 h before the addition of cell-specific media containing 10% untreated FCS or 10% complement inactivated FCS with the addition of 300µg/ml of INT-1, INT-2, EX-1 antiserum and sheep IgG (species isotype control). The cells were harvested at each time point of 1, 2, 4, 6 & 8 days after establishing of cultures and cell number was determined using the automated Coulter counter method. The media from each test and control culture was retained and quantitatively assayed for the presence of HA. The concentration of the HA in the control media (no cell contact) was used to determine the endogenous levels of HA in the growth medium and was subtracted from each subsequent HA determination.

Initial titration of HAS antibodies on MDA-MB-468 and 231 were conducted where cells were incubated in the presence of each antibody for 24-hours after which the cell number was estimated. The titration range of each antibody was 10-10,000ng/mL. A similar trend was observed for each cell line therefore results for MDA-MB-231 are shown.

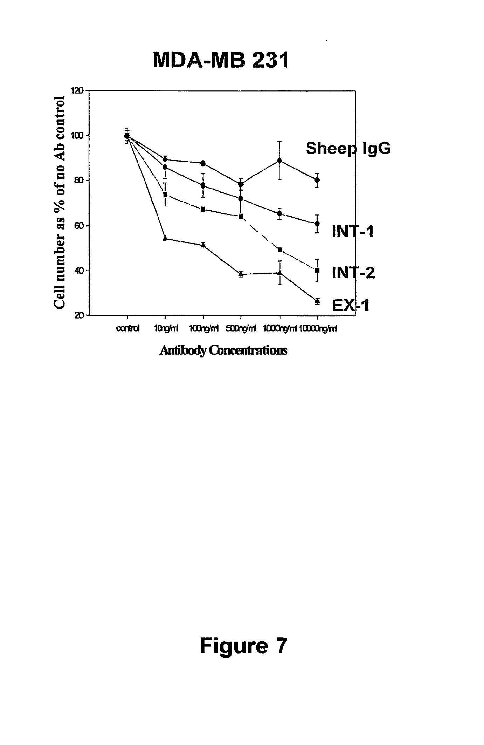

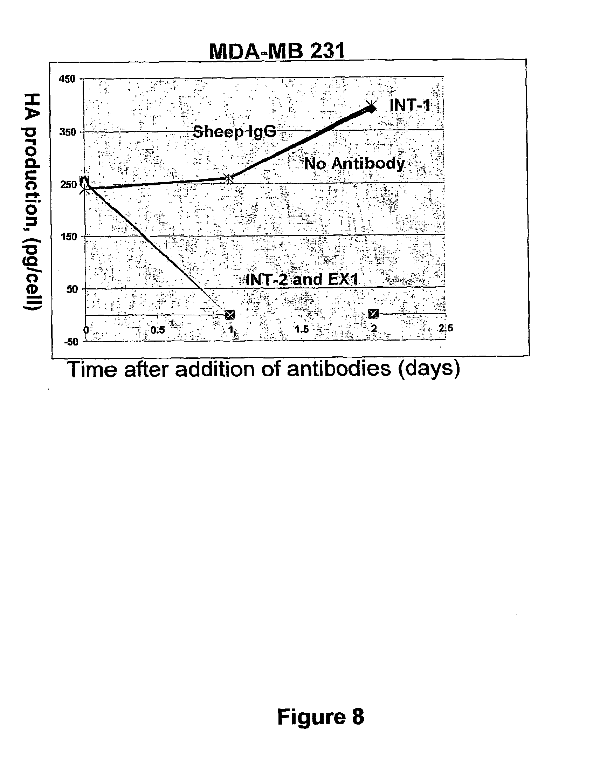

From Figure 7 note that in addition to the antibody to the EX-1 epitope, INT-2 inhibited cellular proliferation and caused a marked decrease in cell number. This was an indication that the membrane topology of the hyaluronan synthase in the malignant state is different to that observed in non-malignant cells such as dermal fibroblasts. These observations translated to functional relevance when additional experiments were performed where both breast cancer cell lines were incubated with 300µg/mL of each antibody. Cells were harvested at each time point and cell number estimated using an automated coulter counter. The media from each test and control culture was retained then assayed for the presence of HA. Figure 8 clearly demonstrates that in addition to EX-1, the HA production in both breast cancer cell line tested was inhibited also by INT-2, but not INT-1.

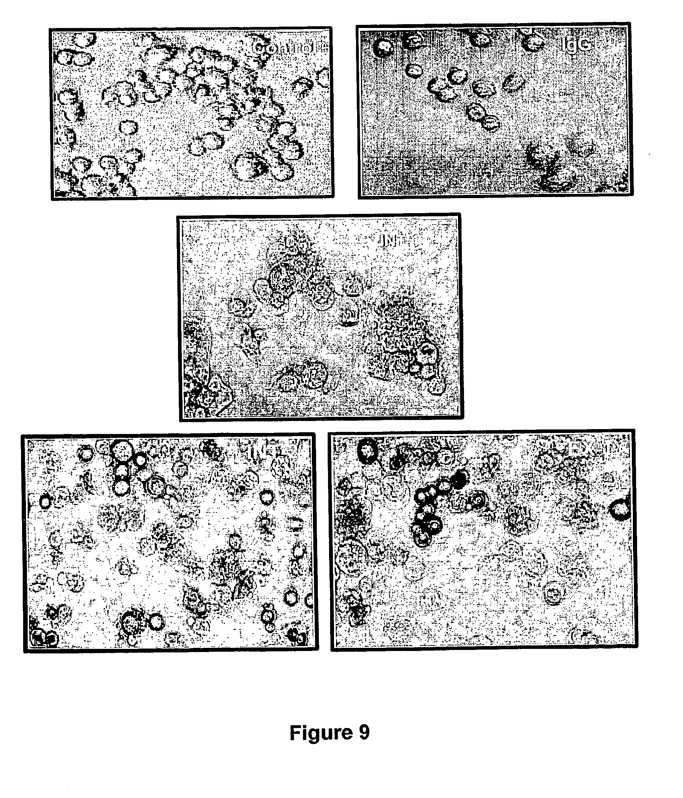

Inhibition of HA synthesis resulted in detachment of viable breast cancer and non-malignant fibroblasts. Microscopic examination of the cultured cells showed distinct morphological differences between cells, 24h after addition of the antibodies. The antibodies to the different synthase epitopes resulted in varied cellular changes. For example the antibody to INT-1 did not exert any dramatic changes in cell morphology. The antibodies to INT-2 and EX-1 resulted in features like cell shrinkage, formation of membrane bound vesicles, and extensive blebbing of the plasma and nuclear membrane. Images captured from MDA-MB-231 grown in the presence of each antibody are shown in Figure 9.

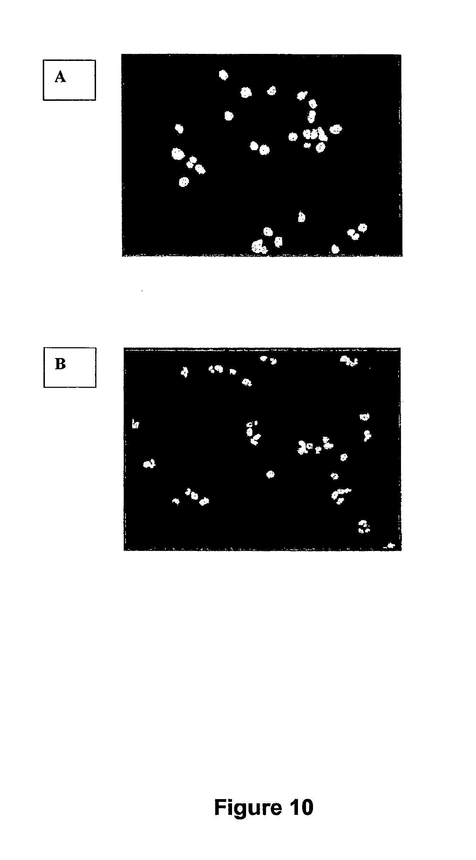

Note the appearance of cells grown in the presence of INT-2 and EX-1 where swollen cells are clearly visible as well as nuclear membrane swelling. It was of logical direction to ascertain the state of the viability of these cells. DAPI staining (to detect nuclear apoptosis) and DNA fragmentation assay were performed. These results are shown below in Figure 10.

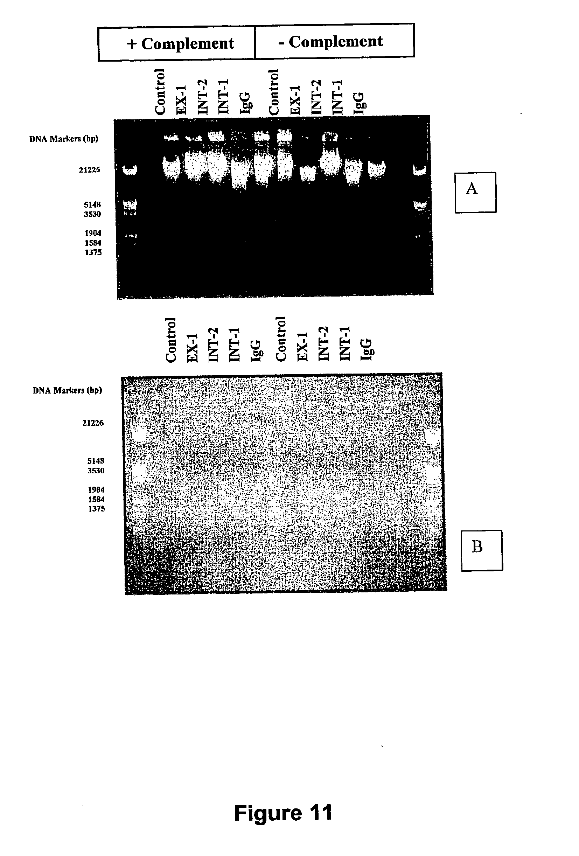

When performing DAPI stains on both detached and attached cells grown in the presence of the HAS antisera, the nucleus did not exhibit and DNA fragmentation (Figure 10; panels A and B). This observation was also confirmed when DNA was extracted from these cells and resolved under standard electrophoretic techniques. DNA fragmentation was not observed (Figure 11; panels A and B).

To confirm the cellular reactivity of the HAS antisera to breast cancer cell lines, FACS was employed in an identical fashion when dermal fibroblasts were initially characterized. Two additional breast cancer cell lines, MDA-MB-435 and ZRL-75-1 were also assessed for reactivity to the HAS antisera.

Note: Where histograms are presented with grey filled plots this represents the reactivity to the HAS antibody and the unfilled plot the corresponding antibody control. Where plots are presented in black lines these represent the background fluorescence as measured from the secondary-FITC only control. The green plots represent the reactivity of each HAS antibody to the cited breast cancer cell lines. The FACS data derived from the 4 breast cancer cell lines tested strongly suggest that in the malignant state the epitope for EX-1 and INT-2 are expressed extracellularly.

### EXAMPLE 5

### Cross reactivity of the HAS antisera with different HAS isoforms

Real-time RT-PCR has been used to characterise the mRNA level for each of the aforementioned breast cancer cells lines. Comparative RT-PCR in conjunction with Northern blot analysis has also been performed to characterise HAS isoform expression in dermal fibroblasts. These results are summarised in Table 3.

**Table 3: Quantitation of HAS isoform expression and reactivity to HAS antisera in malignant and non-malignant cells. HAS expression for breast cancer cell lines is expressed as the fold difference relative to the least invasive cell line (MDA-MB-453). Dermal fibroblast northern blot data demonstrate high transcript levels for HAS2 and very low levels for HAS3. ND: not detected**

| **Cell line** | **Synthase gene expression** | | | **Reactivity with HAS antisera (FACS data)** | | |
|---|---|---|---|---|---|---|
| | HAS1 | HAS2 | HAS3 | EX-1 | INT-1 | INT-2 |
| MDA-MB-231 | ***ND*** | ***15*** | ***1*** | ***YES*** | ***NO*** | ***YES*** |
| MDA-MB 468 | ***ND*** | ***0.1*** | ***3*** | ***YES*** | ***NO*** | ***YES*** |
| MDA-MB 435 | ***ND*** | ***0.5*** | ***0.5*** | ***YES*** | ***NO*** | ***YES*** |
| ZRL-75-1 | ***ND*** | ***ND*** | ***0.5*** | ***YES*** | ***NO*** | ***YES*** |
| Dermal Fibs | ***ND*** | ++++ | + | ***YES*** | ***NO*** | ***NO*** |

### BIBLIOGRAPHY

Antibodies: A Laboratory Manual, Harlow and Lane (eds.), Cold Spring Harbor Laboratory Press, Cold Spring Harbor, NY, 1988
Delpech et al, J. Int. Med. 242:41-48, 1997
Fulop, Arch. Iochem. lophys. 337:261-266, 1997
Itano & Kimata, Biochem. Biophys. Res. Comm. 222:816-821, 1996
Kennet *et al,* (eds.), Plenum Press, New York, 1980
Knudson et al, The Biology of Hyaluronan 143:150-169, 1989
Knudson & Knudson, FASEB J. 7:1233, 1993
Knudson, Am. J. Pathol 148:1721-1726, 1996
Laurent & Fraser, FASEB J 6:2397-2404, 1992
Rooney et al, Int. J. Cancer 60:632-636, 1995
Ruoslahti & Yamaguchi, Cell 64:867-869, 1991
Toole, J. Internal Medicine 242:35-40, 1981
Udabage et al, Cancer Res 65:6139-6150, 2005
Weissman & Meyer, J. Am. Chem. Soc. 76:1753, 1954

## Claims

1. A therapeutic antibody for use in a method for treatment of cancer, wherein said therapeutic antibody targets an epitope of hyaluronan synthase (HAS), wherein said epitope comprises an amino acid sequence represented by GAYREVEAEDPGRLAVE (INT-2), or a sequence containing one or more conservative amino acid substitutions of INT-2.

2. The therapeutic antibody according to Claim 1 wherein said therapeutic antibody is selected from the group consisting of monoclonal antibodies, polyclonal antibodies, and antigen-binding antibody fragments.

3. The therapeutic antibody according to any one of Claims 1 and 2 wherein the HAS is selected from the group consisting of isoforms HAS I, HAS II and HAS III.

4. The therapeutic antibody according to Claim 3 wherein the isoform is HAS II.

5. The therapeutic antibody according to any one of Claims 1 to 4 wherein the cancer is breast cancer.

6. Use of the therapeutic antibody as specified in any one of Claims 1 to 4 for the preparation of a medicament for the treatment of cancer .

7. Use of the therapeutic antibody according to Claim 6 wherein the cancer is breast cancer.

8. Pharmaceutical composition or formulation comprising the therapeutic antibody according to Claim 1 to 4 for use in the treatment of cancer .

9. Pharmaceutical composition or formulation according to Claim 8 for use in the treatment of cancer associatied with HA wherein the cancer is breast cancer.

## Patentansprüche

1. Therapeutischer Antikörper zur Verwendung in einem Verfahren zur Behandlung von Krebs, wobei der therapeutische Antikörper auf ein Epitop der Hyaluronan-Synthase (HAS) zielt, wobei das Epitop eine Aminosäuresequenz, die durch GAYREVEAEDPGRLAVE (INT-2) oder eine Sequenz, die eine oder mehrere konservative Aminosäuresubstitionen von INT-2 enthält, dargestellt ist, umfasst.

2. Therapeutischer Antikörper gemäß Anspruch 1, wobei der therapeutische Antikörper ausgewählt ist aus der Gruppe bestehend aus monoklonalen Antikörpern, polyklonalen Antikörpern und Antigen-bindenden Antikörperfragmenten.

3. Therapeutischer Antikörper gemäß einem der Ansprüche 1 und 2, wobei HAS ausgewählt ist aus der Gruppe bestehend aus den Isoformen HAS 1, HAS II und HAS III.

4. Therapeutischer Antikörper gemäß Anspruch 3, wobei die Isoform HAS II ist.

5. Therapeutischer Antikörper gemäß einem der Anspruch 1 bis 4, wobei der Krebs Brustkrebs ist.

6. Verwendung des therapeutischen Antikörpers, wie in einem der Ansprüche 1 bis 4 beschrieben, zur Herstellung eines Medikaments für die Behandlung von Krebs.

7. Verwendung des therapeutischen Antikörpers gemäß Anspruch 6, wobei der Krebs Brustkrebs ist.

8. Pharmazeutische Zusammensetzung oder Formulierung, die den therapeutischen Antikörper gemäß Anspruch 1 bis 4 zur Verwendung für die Behandlung von Krebs umfasst.

9. Pharmazeutische Zusammensetzung oder Formulierung gemäß Anspruch 8 zur Verwendung für die Behandlung von Krebs, der mit HA im Zusammenhang steht, wobei der Krebs Brustkrebs ist.

## Revendications

1. Anticorps thérapeutique à utiliser dans un procédé pour le traitement du cancer, dans lequel ledit anticorps thérapeutique cible un épitope d'hyaluronan synthase (HAS), dans lequel ledit épitope comprend une séquence d'acides aminés représentée par GAYREVEAEDPGRLAVE (INT-2), ou une séquence contenant une ou plusieurs substitutions conservatrices des acides aminés de INT-2.

2. Anticorps thérapeutique selon la revendication 1 dans lequel ledit anticorps thérapeutique est sélectionné parmi le groupe se composant d'anticorps monoclonaux, d'anticorps polyclonaux, et de fragments d'anticorps de liaison à l'antigène.

3. Anticorps thérapeutique selon l'une quelconque des revendications 1 et 2 dans lequel l'HAS est sélectionné parmi le groupe se composant des isoformes HAS I, HAS II et HAS III.

4. Anticorps thérapeutique selon la revendication 3 dans lequel l'isoforme est l'HAS II.

5. Anticorps thérapeutique selon l'une quelconque des revendications 1 à 4 dans lequel le cancer est un cancer du sein.

6. Utilisation de l'anticorps thérapeutique tel que spécifiée dans l'une quelconque des revendications 1 à 4 pour la préparation d'un médicament pour le traitement du cancer.

7. Utilisation de l'anticorps thérapeutique selon la revendication 6 dans laquelle le cancer est un cancer du sein.

8. Composition ou formulation pharmaceutique comprenant l'anticorps thérapeutique selon la revendication 1 à 4 à utiliser dans le traitement du cancer.

9. Composition ou formulation pharmaceutique selon la revendication 8 à utiliser dans le traitement des cancers associés au HA, où le cancer est un cancer du sein.
